# EUROPEAN PATENT APPLICATION

(11) **EP 3 121 524 A1**
(43) Date of publication of application: **25.01.2017**
(21) Application number: 15765149.8
(22) Date of filing: 17.03.2015
(51) Int. Cl.: F24F 3/16

(54) **AIR PURIFICATION DEVICE**

(30) Priority: 17.03.2014 CN 201420119487 U
(71) Applicant: Law, Sui Chun, Hong Kong (CN)
(72) Inventor: LAW, Sui Chun, N.T. Hong Kong (CN)
(74) Representative: Becker Kurig Straus
(86) International application number: PCT/CN2015/074373
(87) International publication number: WO 2015/139606

(57) **Abstract**

An air purification device comprising: air flow path adjuster (10) and a primary filter (30), the said air flow path adjuster (10) able to change the path of the air flow within the air cleaning device, so that to enable the air bypassing or passing through said primary filter (30). The air purification device can be improved air cleaning efficiency.

## Description

### Background

Pollutants are mainly composed of two forms, one is particulate type pollutants such as dust, bacteria, mold, whose shape is large and of complex molecular structures. It is combined by a variety of different materials or ingredients that, with the dimension ranges from 0.01 micron meters to several hundred micron meters. Another is the gaseous type pollutants. Examples of them included those gaseous type odor and those volatile organic compounds like chemical compounds. It has simple chemical structure. It consists of a variety of chemical elements. The dimension of it is small and ranges in a scales of angstroms to nanometers.

Generally to remove the dust or larger size particulate type pollutants, the electrostatic precipitator or the high-performance dust filter dust will be employed. The dust removal efficiency can be achieved to 80% or even more to 99.9%. However, it is not an easy case to handle the volatile organic compounds which are of the scale from angstroms to nanometers.

Usually in such devices, the two major forms of air pollutants (particulate and gaseous pollutants) are being drawn into the system together. The polluted air first would pass through the particulate removal filter. It would then subsequently enter to the gaseous removal filter. Even a very high performance particulates removal filter could not remove the particulates from the air up at hundred percent perfectly.

In adverse and heavy polluted areas, such as smoking area, the drawn-in polluted air into the system containing particulate removal filter and gaseous removal filter would not completely remove the particulates pollutants. As a result, the un-filtered particles will be deposited on the surface of the gaseous removal filter, blocking the surface of the adsorption sites for gaseous removal. As a result, the gaseous phase pollutants cannot be adsorbed and be eliminated effectively. The accumulation of the particle phase pollutants on the surface of the gaseous removal filter could lead to other problems, such as the filter will provide an incubation area for the growth of bacteria. The turning on these air purification system, which contain the contaminated gaseous removal filter, would release the originally collected pollutants (e.g, bacteria, odor, dust etc.) from the device.

Moreover, the mechanism of filter and purify the gaseous type pollutants and particulate type pollutants are generally different. When the particulate type pollutants are filtered, it is just like using a sieve. When a particulate removal filter is employed for filtering the particulate type pollutants, the faster the air flow rate it is, the more can particulate type pollutants will be separated and filtered, the more obvious effects it will be. However, the purification mechanism of gaseous type pollutants are by either adsorption or catalytic decomposition chemical reaction. It is first required for the gaseous type pollutants be adsorbed onto the surface of the filter materials. If air flow rate is fast, there will not have sufficient residence time for the gaseous type pollutants to be adsorbed on the materials surface of the gaseous removal filter. On another hand, if the airflow rate is slow, the air purification device cannot purify the gaseous pollutants from the environment in short time.

### Summary of the Invention

In view of this, it is necessary to provide a kind of high efficiency air purification device.

An air purification device, comprising an air flow path adjuster and a primary filter; the air flow path adjuster means to change the path of the air flow within the air purification device, so that the air passing through or bypass the primary filter.

In one embodiment, the primary filter is a filter for removing gaseous pollutants, the air is adjusted to flow at a slower rate when it is to pass through the primary filter, comparing to when it is to bypass the primary filter; or the primary filter is a filter for removing particulate pollutants, the air is adjusted to flow at a faster rate when it is to pass through the primary filter, comparing to when it is to bypass the primary filter.

In one embodiment, the air purification device further comprises a secondary filter, the primary filter and the secondary filter are placed separately with space.

In one embodiment, the air flow path adjuster and the primary filter is in a flexible connection. According to a preset sequence, the air is designed to pass through the primary filter and then the secondary filter. The air flow path adjuster is moved or rotated relative to the primary filter, so as to adjust the air flow rate, and enable the air to bypass the primary filter.

In one embodiment, the air flow path adjuster is connected to the primary filter so that it can be rotational coupled onto the primary filter. The air flow path adjuster overlap on the air-in side of the primary filter, and enable to air to bypass the primary filter.

In one embodiment, the air flow path adjuster fixedly connected to the primary filter, or the air flow path adjuster and the primary filter is formed integrally by a same materials. The air flow path adjuster and the primary filter rotate integrally to adjust the air flow rate, and enable to air to bypass the primary filter.

In one embodiment, the air purification device further comprises a fan, the fan is disposed between the primary filter and the secondary filter. The fan enable to air to accelerate and flow from the primary filter or the air flow path adjuster to the secondary filter. The air purification device can adjust the airflow rate of the fan, so as to match the air flow path adjuster.

In one embodiment, the air purification device further comprises a fan, the fan is disposed adjacent to the primary filter, and away from one side of the secondary filter, or it is disposed to adjacent to the secondary filter, and away from one side of the primary filter. The air purification device can adjust the airflow rate of the fan, so as to match the air flow path adjuster.

In one embodiment, the fan is capable to generate a negative pressure, so as to enable the air to pass through the secondary filter and the air flow path adjuster as its preset order.

In one embodiment, the air purification device further comprises a negative ion generator; the ion generator comprises an electronic circuit and an ion releasing tip; and the ion releasing tip is disposed with some space from the primary filter, so as to enable the air be first passing through the ion releasing tip then subsequently the primary filter.

In one embodiment, the air purification device is formed with one air inlet and two air outlets, the primary filter and the secondary filter are placed at the two air outlets respectively, so as to enable the air be split to pass through the primary filter and the secondary filter at the same time, and the air flow path adjuster is flexibly connected to the primary filter, the air flow path adjuster can be moved relative to the primary filter or turn to rotate to adjust the flow rate of air, and enable to air to bypass the primary filter.

In one embodiment, the air purification device further comprises a fan, the fan is disposed adjacent to the air inlet, and speed up the air to flow towards the two air outlets. The air purification device can adjust the airflow rate of the fan, so as to match the air flow path adjuster.

In one embodiment, the air purification device further includes a particulate sensor and a central processing unit, the central processing unit is electrically connected to the fan and the air flow path adjuster. According to the concentration of particulate pollutants and/or gaseous pollutants, the central processing unit control the air flow path adjuster, so that the air flow path adjuster will partially be or completely be opened or closed; the central processor would control the speed of the fan to match the air flow path adjuster.

In one embodiment, the air purification device further comprises a dust sensor and a volatile organic compound sensor; the dust sensor and the volatile organic compound sensors are connected electrically to the central processing unit; when the dust sensor detected that the dust in the ambient concentrations is higher than the default value, the central processor will control the air flow path adjuster so that the air will bypass the primary filter and flow directly through the secondary filter; the volatile organic compound function to detect the concentration of the volatile organic compound surrounding the air purification device; Bases on the concentration of volatile organic compounds in the surrounding environment, the time required for such gaseous pollutants to be adsorbed into the gaseous removal filter, and the static pressure that can be withstand by the gaseous removal filter, the central processing unit regulate the airflow speed of the fan.

When one embodiment, the primary filter is an electrostatic precipitator, when the cumulative amount of dust exceeded the preset level which could be hold by the primary filter, the central processing unit of the air purification device gives out warning signal.

In one embodiment, the primary filter and the secondary filter is disposed in a parallel manner, the air pass through the primary filter as a laminar flow, the air pass through the secondary filter as turbulence flow.

In one embodiment, the primary filter is a gaseous removal filter for removing the gaseous pollutant and the secondary filter is a particulate removal filter for removing the particulate pollutants; or the primary filter is a particulate removal filter for removing the particulate pollutants and the secondary filter is a gaseous removal filter particulate removal filter for removing gaseous pollutant; or the primary filter is for removal of both particulate and gaseous pollutants, whereas the size of the particulate pollutants to be removed by the primary filter will be smaller in compared to that to be removed by the secondary filter.

In one embodiment, the primary filter is for removing particulate pollutants in a dimension of 2.5 microns or smaller, the secondary filter for removing larger particulate pollutants, which are in a dimension of equal to 10 micron or larger.

In one embodiment, the secondary filter is an electrostatic precipitator or high efficiency dust filter.

In one embodiment, the primary filter comprises a first filter material layer and the secondary filter material layer for the air to pass through. The first filter material layer is a filter layer for filtering the particulate pollutants, the secondary filter material layer is a filter layer comprises of activated carbon or zeolite-based porous gas adsorbent materials.

In one embodiment, the primary filter further comprises an ultraviolet light and titanium dioxide compounds for catalyze the oxidation and decomposition of organic pollutants in the air.

Since the air purification device can change the path of the air flow to flow within it by the application of the air flow path adjuster, whenever there is a change on air pollution condition, the air can be purified by either selected to pass through or not pass through the primary filter. The time to purify the air becomes faster, and the efficiency of air purification is improved.

### Brief Description of the Drawing

The present invention is illustrated by the preferred drawings herein. With the drawings, the invention, which being described above and its objective, features and advantages will become more apparent. All portions in the drawings, which described by the same reference numbers, are not in its actual size, i.e. the drawing are deliberately scaled. To illustrate the gist of the present invention always be the main aim.
Figure 1 a and Figure 1b are schematic structural view of one embodiment of an air purification device, when it is of two different states of operation;
Figure 2 is a schematic structural view of another embodiment of the air purification device;
Figure 3 is a schematic structural view of another embodiment of the air purification device;
Figures 4a and 4b are schematic structural view of another embodiment of the air purification device, when it is of two different states of operation;
Figure 5 is a schematic structural view of another embodiment of the air purification device;
Figure 6 is a flowchart of the operation of one embodiment of the air purification device;
Figure 7a and 7b are schematic structural view of the air purification device of one embodiment in two different states of operation;
Figure 8 is a schematic structural view of another embodiment of the air purification device;
Figure 9a and 9b are schematic structural view of another embodiment of the air purification device, where the air flow path adjuster are in two different states of operation;
Figure 10a and 10b are schematic structural view of another embodiment of the air purification device, where the air flow path adjuster are in two different states of operation;
Figure 11 a and 11 b are schematic structural view of another embodiment of the air purification device, where the air flow path adjuster are in two different states of operation;
Figure 12a and 12b are schematic structural view of another embodiment of the air purification device, where the air flow path adjuster are in two different states of operation;
Figure 13a and 13b are schematic structural view of another embodiment of the air purification device, where the air flow path adjuster are in two different states of operation;
Figure 14 is structure diagram of a conventional bladeless fan;
Figure 15 is a structural diagram of one embodiment of the air purification device;
Figure 16a is a partial schematic view of the air purification device as illustrated in Figure 15
Figure 16b showing a partial enlarged view of the air flow path adjuster as illustrated by Figure 16a;and
Figure 16c illustrates a partially enlarged view of another operation status of the air flow path adjuster by the air purification device as shown Figure 16a.

### Detailed Description of the Embodiments

To make the above-mentioned objectives characteristic features and the advantages of the present invention be more easily to be understood, the following are some detailed description of the embodiments of the present invention. The following description contains numerous specific details in order to enable the present invention to be fully understood. However, the present invention can be implemented in many other ways, other than those described herein. The person of original skill in the art can make similar improvements without departing from the connotation of the invention. The invention is not limited by the following disclosed specific embodiments.

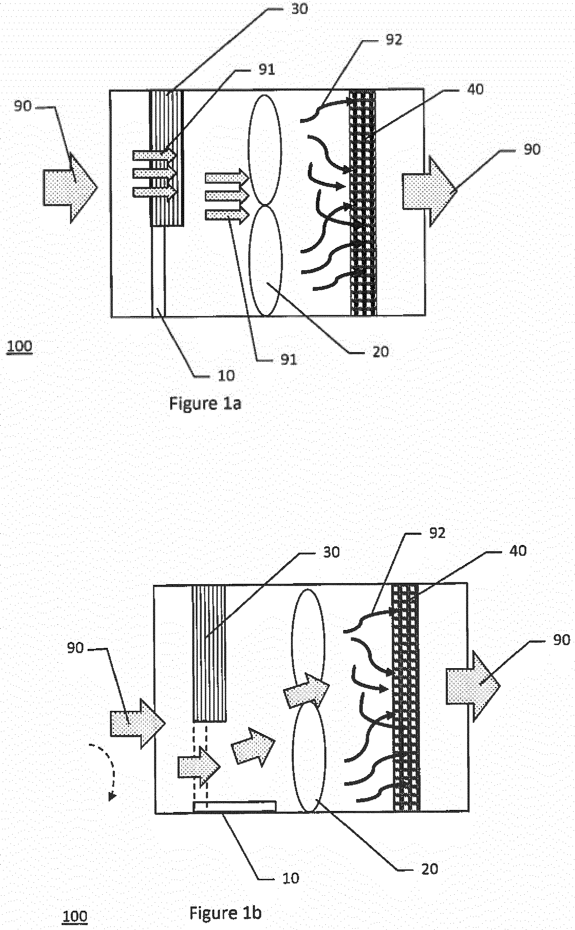

As shown in embodiment as illustrated in Figure 1 a and 1b, the air purification device 100 comprises an air flow path adjuster 10, a blower 20, the primary filter 30 and a secondary filter 40. The air flow path adjuster and the primary filter 39 are disposed in a parallel manner, the secondary filter 40 and the primary filter 30 are disposed with some space apart, and the fan 20 is disposed in between the primary filter 20 and the secondary filter 40.

In particular, the fan 20 is provided at a position downstream of the primary filter 30 and the air flow path adjuster 10, it is being placed in the upstream position of the secondary filter 40. The air inlet of the fan 20 is connected to the primary filter 30 and the air flow path adjuster. The air outlet of the fan 20 is connected to the secondary filter 40. When the air flow path adjuster 10 is in a closed position (or as shown in the Figure 1a, the air flow path adjuster 10 is being adjusting to a position such that it allow the air to pass through both the primary filter 30 and secondary filter 40). The air 90 is being drawn into the primary filter 30, and within the materials of the primary filter 30, in a laminar flow 91 is formed. Then, the air is blown onto the secondary filter 40. When the air is drawn to flow through the secondary filter 40, it is flow in a form of turbulence flow 92. When the air flow path adjuster 10 is in an opened position (or as shown in the Figure 1b, the adjuster 10 is being adjusting to a position that the air 90 bypass the primary filter 30, i.e., the air 90 does not pass through the primary filter 30 but pass through only the secondary filter 40 only). The air is being drawn into the air purification device 100 where it is then drawn to blow onto the secondary filter 40. When the air is passing through the secondary filter 40, it is flow in a form of turbulence flow 92.

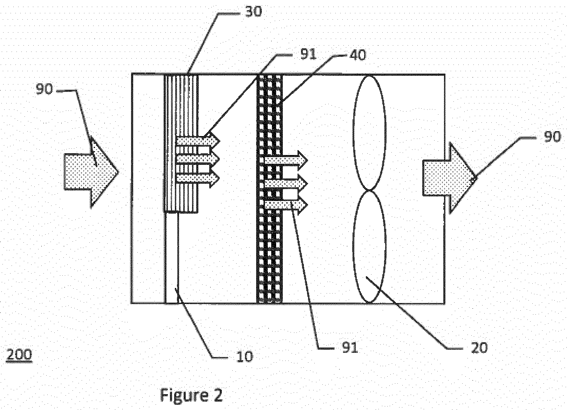

As indicated in the embodiment of an the air purification device 200 as shown in the Figure 2, the fan 20 is provided at a downstream position of the primary filter 30 and secondary filter 40. The air inlet of the fan 20 is connected to the primary filter 30 and the air flow path adjuster 20, or the secondary filter 40. Further, when the air flow path adjuster 10 is turned to closed position (it is adjusted to a position so that air can flow through both the primary filter 30 and the secondary filter 40 at the same time), the air 90 is drawn into the primary filter 30 and the secondary filter40, the air is flowing as a laminar flow 91.

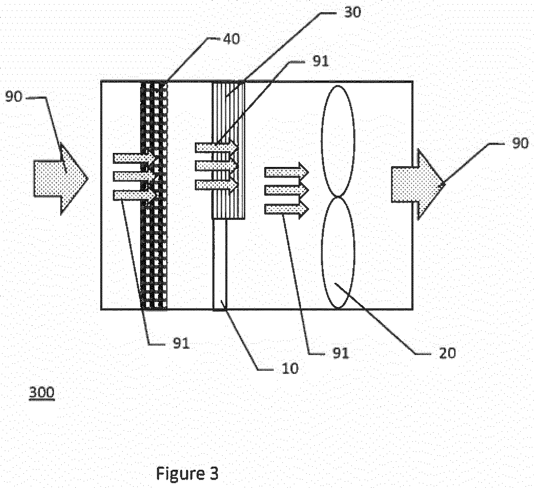

As indicated in the embodiment of an air purification device 300 as shown in the Figure 3, the primary filter 30 is disposed downstream of the secondary filter 40, the fan 20 is disposed downstream of the primary filter 30 and secondary filter 40. When the air flow path adjuster 10 is turned to closed position (it is adjusted to a position so that air can flow through both the primary filter 30 and the secondary filter 40 at the same time), the air 90 is drawn into the first filter 30 and the secondary filter40, the air is flowing as a laminar flow 91.

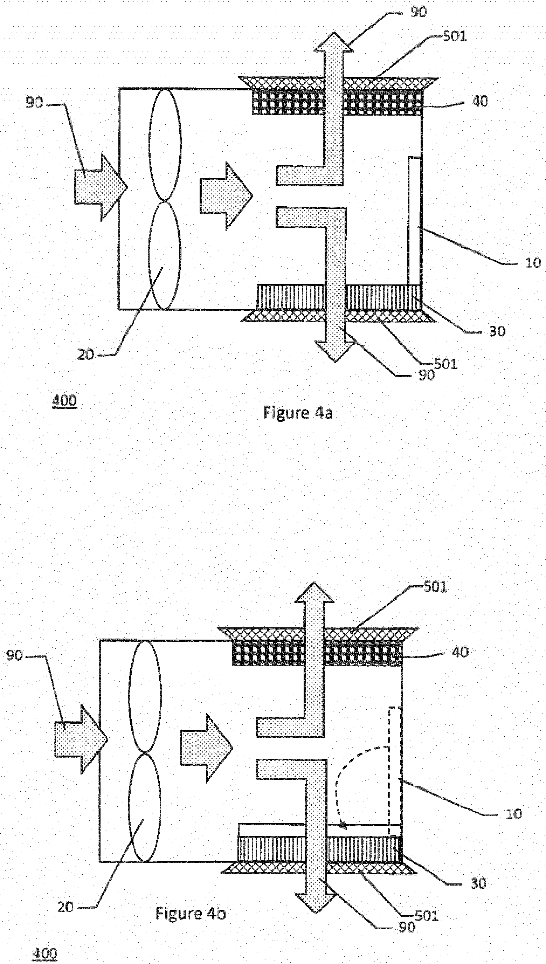

As shown in Figures 4a and 4b, the air purification device 400 of another embodiment, comprises one air inlet and at least two air outlets 501. The two outlet 501 is provided with a primary filter 30 with an air flow path adjuster 10, and a secondary filter 40 respectively. Wherein, the fan 20 is provided at the upstream position of the primary filter 30 and secondary filter 40, the air outlet of the fan 20 is connected to the primary filter 30 and the air flow path adjuster 10, as well as the secondary filter 40. When the air flow path adjuster 10 is adjusted to a position so that air can flow through both the primary filter and the secondary filter is (shown in Figure 4a), the air 90 is drawn to pass through the primary filter 30 and the secondary filter 40. It is then be discharged from the air outlets of the air purification device 501 respectively. When the air flow path adjuster 10 is adjusted to another position (as shown in Figure 4b, it is adjusted a position so that it is covering the primary filter filter) the air 90 would only pass through the secondary filter 40, but would not pass through the primary filter 30 of the air purification device 400, it is then be discharged from one air outlet 501.

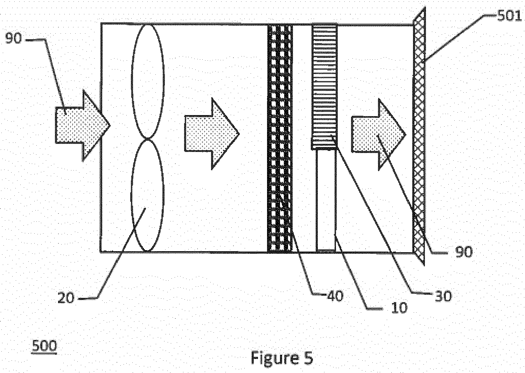

As shown in Figures 5 of an air purification device 500 of one embodiment, the fan 20 is disposed at the upstream position of the primary filter 30 and secondary filter 40, the air outlet of fan 20 is connected the primary filter 30 and the air flow path adjuster 30.

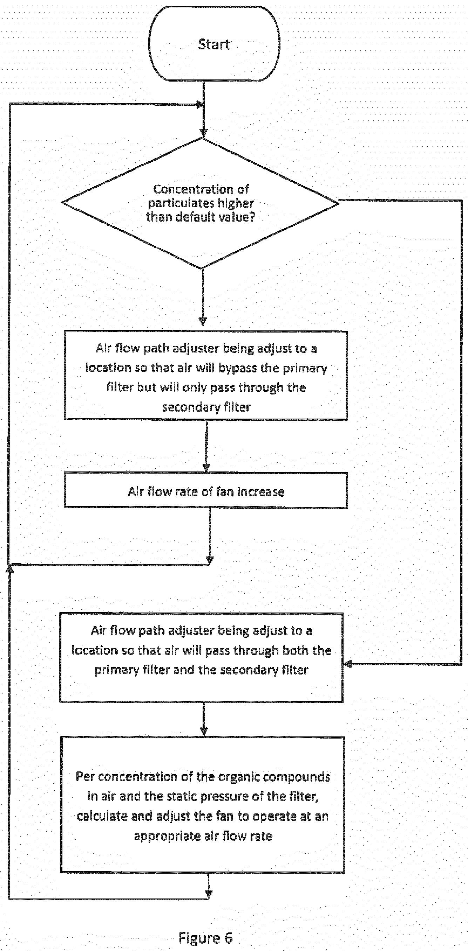

Figure 6 is a flowchart of the turning on and off operation of the airflow path adjuster 10 in one embodiment of the air purification device. Wherein, a primary filter is employed for removing the gaseous pollutants, a secondary filter is employed for removing particulate pollutants. The air purification device further comprises a dust sensor and an organic compounds sensor. The dust sensor detects and measures the concentration of the particulates in the surrounding environment. When the dust concentrations higher than a default value, the air flow rate of the fan will be accelerated and the air flow path adjuster is adjusted to a position so that the air entering the air purification device will skip the primary filter. The air purification devices will continue to operate and draw air from the upstream to downstream positions until the concentration of the particulates reach to a level that is below to the default value. Then, at this instant, the concentration of organic compounds in the ambient air would be collected by the organic compounds sensor. Bases on the concentration of volatile organic compounds in the surrounding environment, the time required for such gaseous pollutants to be adsorbed into the primary filter, and the static pressure that can be withstand by the primary filter, the airflow speed of the fan within the air purification device will be regulated, so that air is cleaned effectively by treating the different type of pollutants at their different levels (concentrations of the organic compound concentration and particulates) intelligently.

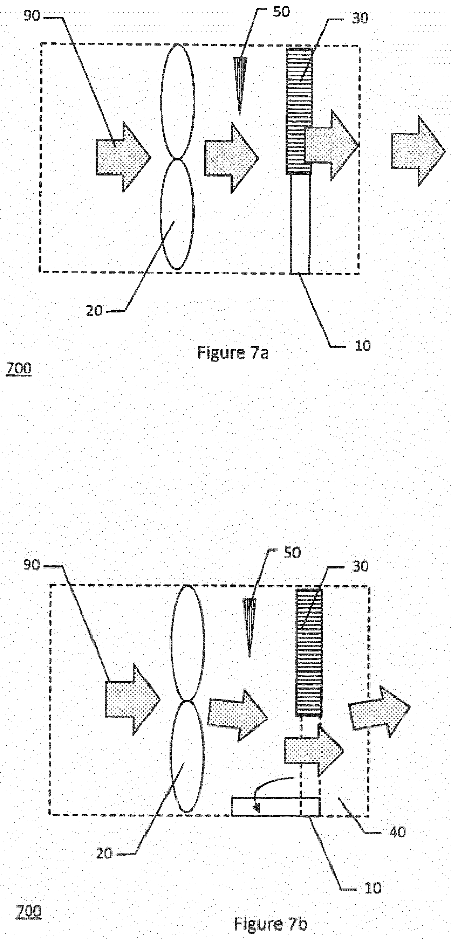

As shown in Figures 7a and 7b of an air purification device 700 of one embodiment, a negative ion generator is further comprised. The negative ion generator comprises an electronic circuit and a negative ion releasing tip 50. The ion releasing tip 50 is placed spaced apart from the primary filter. Shown in Figure 7a, the air flow path adjuster 10 can be adjusted to position such that the air 90 within the air purification device, is able pass through the primary filter 30, i.e. the air flow path adjuster is being adjusted to a position where the air 90 must pass through the primary filter 30. Further, as shown in Figure 7b, the air flow path adjuster can also be adjusted to another position in which the air must bypass the primary filter. In both cases, the air 90 will first passes through the ion releasing tip 50.

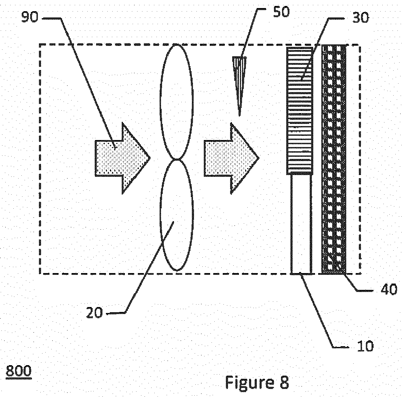

As shown in Figure 8 of an air purification device 800 of one embodiment, a negative ion generator is further comprised. The negative ion generator comprises an electronic circuit and a negative ion releasing tip 50. The ion releasing tip 50 is disposed at an upstream position of the particulate removal filter or the secondary filter 40.

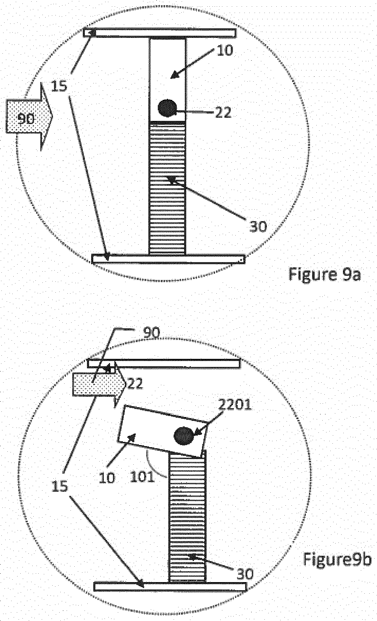

As indicated in the figure 9, an air purification device (with only the air flow path adjuster) being illustrated, the air purification device further comprises a housing 15, the air flow path adjuster 10 being operated to turn on or off by rotating a hinge or a motor 22, so that the air flow path adjuster 10 is adjusted to a closed position (shown in Figure 9a) or to an opened position (shown in Figure 9b). The air flow path adjuster 10 is fixed at one side and adjusted to move at another side. When the air flow path adjuster 10 is wholly or partially opened, it will formed an angle with the primary filter 30 or the gaseous removal filter as described in the present embodiment. The air 90 is forced to discharge directly from the air outlet, which the air outlet is free from air flow resistant.

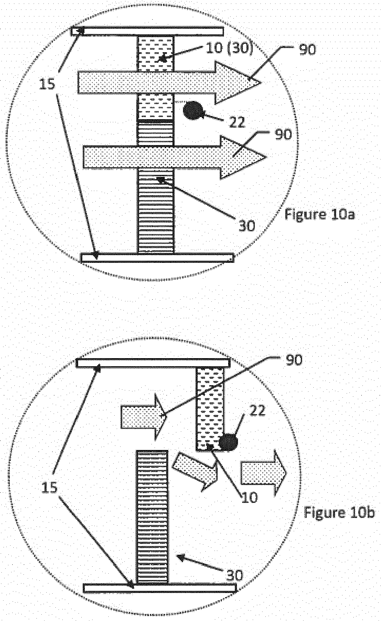

As indicated in the figure 10, an air purification device (with only the air flow path adjuster) being illustrated, the air purification device further comprises a housing 15, the air flow path adjuster 10 being operated to turn on or off by rotating a hinge or a motor 22, so that the air flow path adjuster 10 is adjusted to a closed position (shown in Figure 10a) or to an opened position (shown in Figure 10b). In the case, the whole air flow path adjuster will moved forward or backward. When the air flow path adjuster 10 is wholly or partially opened, it will separate completely from the primary filter 30 or the gaseous removal filter as described in the present embodiment. The air 90 is forced to discharge directly from the air outlet, which the air outlet is free from air flow resistant.

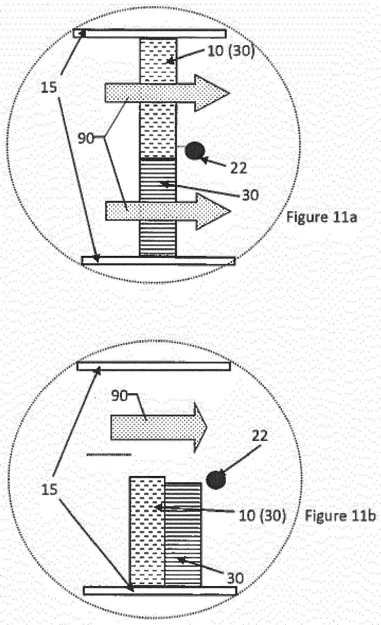

As indicated in the figure 11, an air purification device (with only the air flow path adjuster) being illustrated, the air purification device further comprises a housing 15, the air flow path adjuster 10 (which is also a primary filter 30) being operated to turn on or off by rotating a hinge or a motor 22, so that the air flow path adjuster 10 (which is also a primary filter 30) is adjusted to a closed position (shown in Figure 11 a) or to an opened position (shown in Figure 11b). In the case, the whole air flow path adjuster will be pushed to slide over the primary filter 30. When the air flow path adjuster 10 is wholly or partially opened, it will overlapping the primary filter 30 or the gaseous removal filter as described in the present embodiment. The primary filter 30 or the gaseous removal filter would then have a higher airflow resistant after being overlapped. The air 90 is forced to discharge directly from the air outlet, which the air outlet is free from air flow resistant.

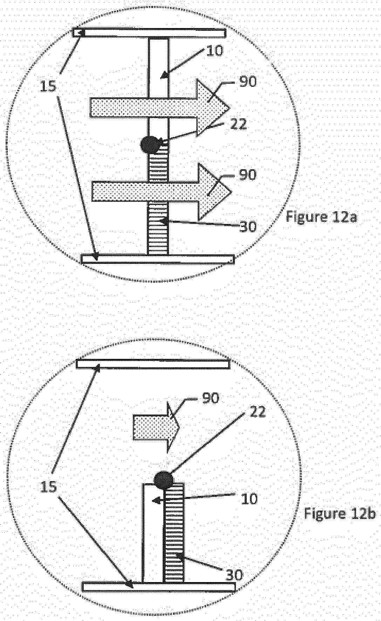

As indicated in the figure 12, an air purification device (with only the air flow path adjuster) being illustrated, the air purification device further comprises a housing 15, the air flow path adjuster 10 being operated to turn on or off by rotating a hinge or a motor 22, so that the air flow path adjuster 10 is adjusted to a closed position (shown in Figure 12a) or to an opened position (shown in Figure 12b). In the case, the whole air flow path adjuster will moved slide over the primary filter. When the air flow path adjuster 10 is wholly or partially opened, it will completely overlapped on the air inlet side of the primary filter 30 or the gaseous removal filter as described in the present embodiment. This will further protect the primary filter from being contaminated by the air 90 with the high concentration of particulates.

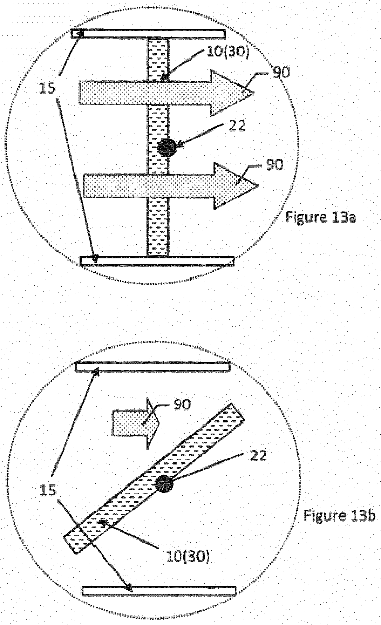

As indicated in the figure 13, an air purification device (with only the air flow path adjuster) being illustrated, the air purification device further comprises a housing 15, the air flow path adjuster 10 and the primary filter 30 is formed integrally by a same materials. In this embodiment, both the air flow path adjuster 10 and the primary filter 30 is functioned to remove the gaseous pollutants. When the air flow path adjuster 10 is adjusted a closed position (shown in Figure 13a) or to an opened position (shown in Figure 13b), the airflow direction of the air 90 is controlled to flow within the air purification device by the airflow adjuster 10, so that when the air flow path adjuster is opened, the air 90 will not pass through the primary filter 10, 30.

When the concentration of dust in the air is higher than the default value, the air flow adjuster will open (shown in Figure 13b), the air passes through the secondary filter (or the negative ion releasing tip or the particulate removal device), and be discharged directly from the air outlet of the air purification device, which the air outlet is free of air flow resistant. The dust and particulates in air will first be removed. It will be until the particulate concentration lower than the default value, the air flow path adjuster 10 will then turn to a closed position (it will be adjusted to a position so that air can flow through both the primary filter and the secondary filter or negative ion releasing tip or particulate removal device), so that gaseous pollutants can also be removed.

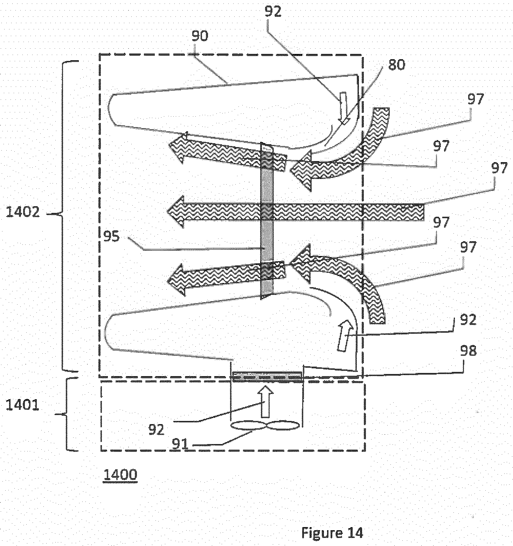

Figures 14 to 16 are some embodiments of the air purification device with its operation examples. The embodiments illustrated that even a device with no air filtration function, can also be transformed to an air purification device and perform air purification to remove gaseous pollutants and particulate pollutants in an order manner.

Figure 14 shows a bladeless fan 1400, which unlike the traditional electric fans, dehumidifiers, humidifiers, air cooling units, air conditioners, heaters and other electrical devices that including a fan, and could be easily turned into air purification device by sampling adding a primary filter on the air flow path that generated by the fan. The bladeless fan is designed to amplifiers the airflow by rely mainly on two air flow paths, in order to achieve an air ventilation effect: (1) an air flow path which is generated by the high torque motor 91, the air being generated is having an air flow high pressure (the primary air flow) 92; as shown in the lower position 1401 of the bladeless fan 1400 as illustrated in Figure 14, the air is flow within the housing. If a filter 98 is added on the air flow path of this high pressure air 92 (the primary air), it will weaken the pressure of this high pressure air 92 (the primary air), if the primary air if of lower pressure and being discharged from the venturi outlet 80. It cannot achieved an effect to entrain the surrounding air 97. (2) Another air flow paths is the air flow by the Coanda effect at the hollow circular housing 90 (as indicated in the upper portion 1402 of the bladeless fan 1400), which drives the surrounding air 97 (the secondary air flow) to flow through the hollow circular housing. If a filter 95 is added on this air flow path, then the shape of a hollow circular housing is changed. The air flow due to the Coanda effect will disappear due to the change of this appearance. The surrounding air 97 (secondary air flow) will not be entrained to flow. These air amplifiers or bladeless fan indeed was very good air ventilator, however, it cannot become an air purification device. It cannot be diverted to become an environmental cleaning device.

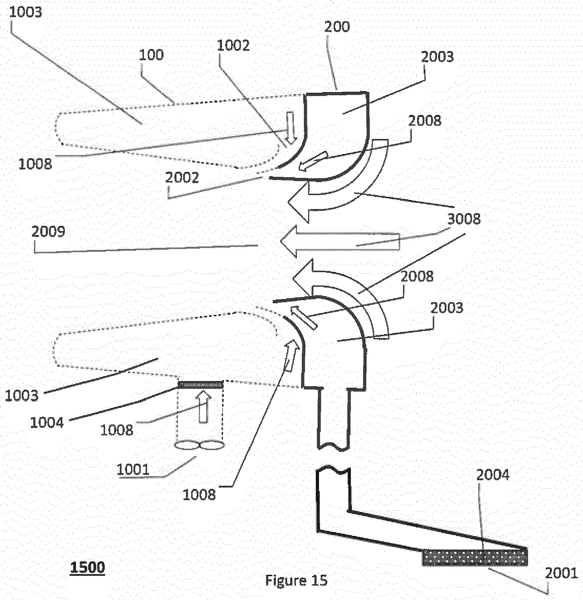

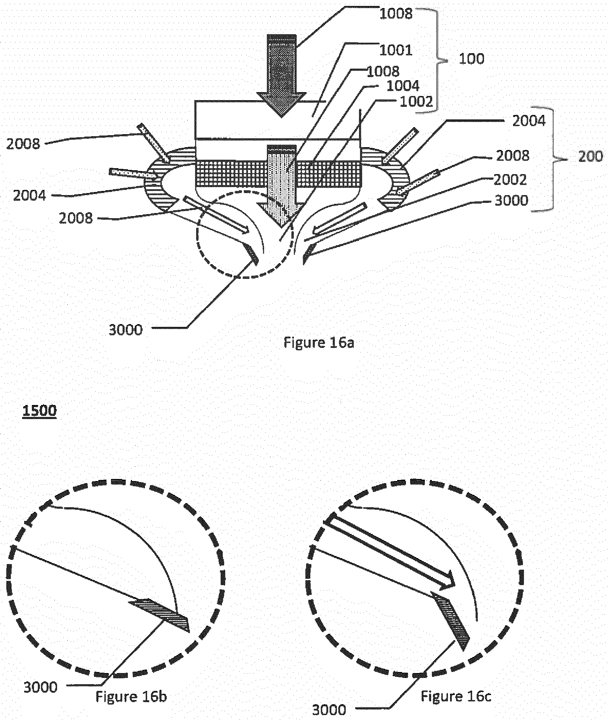

The above air purification device does not required a fan. With the air purification method or device as indicated in the present invention, the bladeless fan can be transformed to an air purification device (as shown in Figure 15). Figure 15 is a side perspective view of an air purification apparatus of the embodiment. Figure 16 is a partial schematic view of an air cleaning device which shown in Figure 15. This transformation can be achieved by:

An air purification device, comprising: at least one primary air flow system 100, a secondary air flow system 200, at least one air flow path adjuster, a primary filter 2004 and a secondary filter 1004.

The primary air flow system 100 comprises at least one housing, at least one primary air inlet 1001, at least one primary air outlet 1002, a primary chamber 1003 which is located between the primary air inlet 1001 and the primary air outlet 1002. At least one fan to drive the primary air flow 1008 to flow within the primary chamber 1003, from the upstream to the downstream position, flows from the primary air inlet 1001 to the primary air outlet 1002, and be discharged from the primary air outlet 1002. The secondary filter 1004 is disposed within the first chamber in 1003, when the primary air flow 1008 is flowing within the primary chamber 1003, it is flowing from the upstream position to the downstream positions, it is flowing through the secondary filter 1004. Wherein, the secondary filter 1004 is a filter with very low airflow resistance. Further, the secondary filter 1004 may be a negative ion generating device. Further, the secondary filter 1004 may be a electrostatic precipitators.

The secondary air flow system 200 comprises at least one housing, at least one primary filter 2004, at least a secondary air inlet 2001, at least one secondary air outlet 2002, and at least a secondary chamber 2003. The primary filter 2004 is provided within the housing. The secondary air inlet 2001 is located at the most upstream position of the housing. The secondary outlet 2002 is located at the most downstream position of the housing. The primary filter 2004 is located at a downstream of the secondary air inlet 2001. The secondary chamber is located between the primary filter 2004 and the secondary air outlet 2002. The primary filter 2004 purify all the air 2008 flow enter to the secondary air flow system 200 through the secondary air inlet 2001, which also pass through the primary filter 2004.

The secondary air outlet 2002 of the secondary air flow system 200 is set adjacent to or slightly upward (slightly upstream) to the primary air inlet 1002 of the primary air flow system 100.

The air flow path adjuster 3000 is disposed a frontier or post- position of the secondary air outlet 2002 of the secondary air flow system 200. So that the secondary air outlet 2002 become an air outlet without obstacle by adjusting the air flow path adjuster 3000. The air flow path adjuster 3000 can also be adjusted to another position so that it closes the secondary air outlet 2002.

The air flow path adjuster 3000 can also be adjusted to aperture with different size, so that the volume and air flow rate of the air 2008 that pass through the primary filter 2004 into the air 2004 can subsequently be adjusted.

When the air (the primary air flow) 1008 in the primary gas flow system is drivento flow from upstream to downstream, the primary air flow from the first air inlet 1001 into the first chamber 1003, then pass through the secondary filter 1004, and then direct be discharged from the first outlet 1002. The polluted air 1008, together with the primary air flow, go through the secondary filter 1004, and being purification firstly. It is until the concentration of pollutants up to the desired level, the air flow path adjuster 3000 will adjust to a position where the secondary air outlet 2002 will become an air outlet without any obstacle. When primary air flow 1008 is being discharged from the first outlet 1002, it will make air adjacent to or slightly upstream position to the primary air outlet 1002, i.e. the air near the secondary air outlet 2002, also the be in traction and be discharged with the primary air flow 1008. A negative air pressure zone is created within the secondary chamber 2003, the secondary air flow 2008 then enter to the secondary air inlet 2001 to the secondary chamber 2003, passing the primary filter 2004 and be further purified. When the secondary air flow 2008 being discharged from the second outlet 2002 by traction flow of the primary air flow 1008, it is flowing out from the secondary air outlet 2002 outflow. It will combined with the primary gas flow 1008 and be is discharged together.

In the present embodiment, the electrostatic precipitator 1004 is employed as the secondary filter 1004, the high efficiency filter (HEPA, High Efficiency Particulate Air) is being employed as the primary filter 2004.

The air purification system 1500 also includes a coarse dust concentration sensor, a fine dust concentration sensor and a central processor. When the fan drives the primary air flow 1008 to flow from the primary air inlet 1001 into the housing of the primary air flow system 100, particulate pollutants of different sizes will flow together with the primary air flow 1008 within the housing. The air is passing through the secondary filter (the electrostatic precipitator) 1004, It is then is emitted from the venturi nozzle 1002. Larger particulate contaminants in the primary flow system 100 is captured by electrostatic precipitator 1004 within the housing, and the first level of air purification is achieved. Meanwhile, with the application of the hollow circular housing appearance to create the Coanda effect, large amount of air 3008 is drawn to flow into the piping of hollow circular housing 2009, an air ventilation effect is also achieved.

On referring to the Figures 16a to 16c, when air flow path adjuster 3000 is adjusted to a position that close the secondary air outlet 2002 (Figure 16b), the air will only pass through and be purified by the secondary filter 1004 (It is because the secondary air outlet 2002 is closed, thus, the air will not pass through the primary filter 2004, as shown in Figure 16b). After some time, when the concentration of coarse dust in the environment is detected to reduced to a desired level by the coarse dust sensor, the central processing unit will then instruction air flow path adjuster 3000 to move to another location. The secondary air outlet 2002 is then opened (as indicated in the Figure 16c), the air flow path adjuster is therefore adjusted to a location which allow the air to flow out from the secondary air outlet 2002, which is an air outlet with no air flow resistance. Since the secondary air outlet 2002 of the secondary air flow system 200 is disposed adjacent to, or slightly upward (slightly upstream) to the primary air outlet 1002 of the primary air flow system 100. When the air is discharged from the primary air outlet 1002, the air at the position adjacent to, or slightly up(slightly upstream) to the primary air outlet 1002, i.e., the air at the secondary air flow outlet 2002, will be flowing in traction to the primary air flow and be discharged together. A negative air pressure is therefore produced within the secondary chamber 2003, a secondary air flow is therefore formed to flow through the primary filter 2004. The air is therefore being further purified. As the first level air purification has already been performed by the secondary air filter, the large particulate pollutants (coarse dust) is at a lower or desired concentration. The coasrse dust particulates, therefore, does not pollute, destroy, or clogging the primary filter 2004. The life of the primary filter 2004 thus being greatly extended.
Since the secondary filter (the electrostatic precipitator) 1004 has a much low air flow resistance, it can quickly reduce the concentration of larger dimension particulate pollutants in the air 1008, and reduce its level to an ideal value. Then, the air with lower level of coarse particulate pollutants will pass through the primary filter 2004, which is of higher air flow resistance. An all-round and more in-depth air filtration is therefore being performed. Compared to the traditional air filtration method where the "air is drawn through all the filters all in a parallel manner", the present air purification device shorten the time to purify the air.

In other embodiments, the secondary filter 1004 is functioned to remove the particulate pollutants, the primary filter 2004 is functioned to remove gaseous pollutants.

Further, when the air flow path adjuster 3000 is adjusted to a position that secondary air outlet 2002 become closed, the air can only be drawn to flow through the secondary filter 1004. The airflow rate of the fan can further be increased, and large volume of air will have it particulates be effectively removed by the secondary filter 1004. When the air flow path adjuster 3000 is adjusted to move another position that the secondary air outlet 2002 becomes opened. The air 1008,2008 will passing through the primary filter 1004 and secondary filter 2004 for air purification. The airflow rate of the fan can be adjust so that a suitable ventilation speed is operated, to draw the air to flow through the secondary filter 1004 and the primary filter 2004 at appropriate speeds.

Air purification system of this invention can intelligently purify the pollutants air separately and orderly according to the dimension of the pollutant. First the larger dimension will be first removed to a desired level, then the finer particulates or even the molecular structure of gaseous pollutants. Compare to the traditional one where all the air is forced to pass through all high efficiency particulate filter and odor filter at once time, which lead to the clogging of the finer particulates filter or odor filter such as the deodorizing filter by the larger size particulate. The present invention provides a highly efficient, environmentally friendly air purification device, when can also reduce secondary pollution.

The air flow rate and air flow volume of the air which pass through the primary filter 2004 (the secondary air flow) is subjected to be controlled by: (1) a surface area of the primary filter 2004; and / or (2) the aperture regulated by the air flow path adjuster 3000. Through the controlling of the area of the primary filter area 2004 and the aperture by the air flow path adjuster 3000, the airflow rate and airflow volume of the air to pass through the primary filter can intelligently control to an optimal value. In this embodiment, the concentration of gaseous pollutants can first be detected by a gas sensor. Then by controlling the air flow path adjuster, the air flow path and volume to flow through the primary filter within the air purification system can be regulated.

This embodiment, there are three air flow path, to achieve the objective of air purification:
The first path is to allow the air to pass through the secondary filter along with the high pressure air flow (the primary air flow 1008) which being created by the high torque motor;

The second path is along with the Coanda effect which created by the hollow circular housing, it drive the surrounding air (secondary air flow 3008) to flow through circular housing. The main function of this air flow path is to enhance the air mobility within the environment. The cleaned air which being discharged from the air purification device shall be delivered to a far distance as soon as possible, whereas the polluted air from the far distance shall be pumped to the air purification device nearby as soon as possible, so that the air can be effectively purified by the air purification device;

The third path is the path by adjusting the air flow path adjuster 10, the secondary air flow is drawn to flow through the primary filter 2008, for the further level of air filtration.

In the present embodiment, a non-traditional way of filter arrangement, where the filters are arranged parallel to the air outlet is executed. The filter is wisely placed within an air amplifier (i.e. the bladeless fan) for effectively purifying the air.

The present invention can have different kind of implementation methods, including rearranging the filters within the device and the position of the air flow path adjuster. As long as the air flow path adjuster is employed to control the air flow path and control the air flow speed within the air purification device; and/or by further controlling the air flow rate and the residence time of the airflow when it is passing through the filters; and when the concentration of the particulates in the air is higher than the default value, the air flow path adjuster will move to open (it is adjusted to a position that the air is by-passing the primary filter and flow through only the secondary filter), then speed of fan is adjusted to accelerate, and the air is drawn to pass flow through the secondary filter, which is a dust removal device, and the air is directly be discharged from the air outlet which is of low airflow resistant; when the airborne dust and particulates is be treated to a concentration which is lower than the default value; the air flow path adjuster will then move to a closed position (it is adjusted to another location so that air can flow through both the primary filter and the secondary filter, which is a negative ion releasing tip or dust removal device), and adjust the ventilation fan speed to go through at least two sets of filter (for removing particulate contaminants and for removing gaseous contaminants), all can effectively remove various type of pollutants. From further Intelligent control of air flow speed, gaseous removal filters can be prevented from being clogged and losing effectiveness.

The air purification device, depends on the filter characteristics to adjusting the air flow path and speed, at the same time it is also depends on the concentration of the pollutants in the environmental, the best cleaning results can be achieved.

The present invention relates to an apparatus and method for purifying air, the air purification device comprises an air flow path adjuster and a primary filter; the air flow path adjuster means to adjust its position within the air purification device, in changing the air flow path within the air purification device, the air may flow to bypass or pass through the primary filter.

When the air purification device further comprises a fan, an air flow path adjuster which adjusting its position within the air purification device. On doing so, the air flow rate of the fan is also being adjusted, the air passes by or passes through the primary filter will be of different air flow rate.

In one embodiment, the primary filter is a filter for gaseous pollutants removal.

In one embodiment, the primary filter is a filter for particulate pollutants removal.

In one embodiment, the primary filter is a filter for removing gaseous pollutants, the air is adjusted to flow at a slower rate when it is to pass through the primary filter, comparing to when it is to bypass the primary filter.

In one embodiment, the primary filter is a filter for removing particulate pollutants, the air is adjusted to flow at a faster rate when it is to pass through the primary filter, comparing to when it is to bypass the primary filter.

In one embodiment, the air flow path adjuster and the primary filter is arranged in a parallel manner.

In one embodiment, the air flow path between the air flow path adjuster and the fan, and the air flow path between the air flow path adjuster and the primary filter, are parallel relationship.

In one embodiment, the air purification device further comprises a secondary filter, the primary filter and the secondary filter are arranged in a parallel manner.

In one embodiment, further comprising a secondary filter, the primary filter with the air flow path adjuster and the secondary filter are arranged in series manner.

In one embodiment, the primary filter is for removing gaseous pollutants, the secondary filter is for removing particulate pollutants.

In one embodiment, the primary filter and the secondary filter is functioned to removing particulate contaminants, wherein the primary filter can remove the finer particulate pollutants, the secondary filter can only remove the larger size particulate pollutants.

In one embodiment, the primary filter is for removing particulate pollutants in a dimension of 2.5 microns or smaller, the secondary filter for removing larger particulate pollutants, which are in a dimension of equal to 10 micron or larger.

With the application of the air purification device and method of the present invention, particulates of different dimension can be removed effectively and orderly. When the concentration of the particulates or the larger size pollutants is higher than a preset value, the fan will be adjusted to flow at a faster rate, the air flow path adjuster will be adjusted so that the air will only pass through the secondary filter, the particulates or the larger size pollutants will first be removed. This can avoid the particulates or the larger size pollutants to be clogged onto the surface of the primary filter, whereas the function of the primary was preliminary designed for removing the smaller sizes particulates or gaseous pollutants (since the primary filter is functioned to remove the smaller size particulate or gaseous pollutants, the cost of it be more expensive than the secondary filter). When a larger size pollutants or the particulates are filtered and be removed so that the level of it is up to a value which would not damage the primary filter, and air flow path adjuster and air flow rate of the fan would then be further adjusted. Then, the smaller pollutants or gaseous pollutants along with the air will pass through the primary filter at an appropriate air flow rate, further purifying the air to remove the smaller size pollutants, or finer particulate pollutants or gaseous pollutants will be carried out.

In one embodiment, the primary filter is a gaseous removal filter for removing the gaseous pollutant and the secondary filter is a particulate removal filter for removing the particulate pollutants. With the turning on and turning off the air flow path adjuster, and with the adjustment of the airflow rate of the fan, the particulate pollutants and the gaseous pollutants in the air will be filtered selectively and orderly.

In one embodiment, the primary filter is a gaseous removal filter for removing the gaseous pollutant and the secondary filter is a particulate removal filter for removing the particulate pollutants. With the turning on and turning off the air flow path adjuster, and with the adjustment of the airflow rate of the fan, the particulate pollutants and the gaseous pollutants in the air will be filtered separately, selectively and orderly. The particulate pollutants will be first be filtered, the subsequently the gaseous pollutants will be filtered.

In one embodiment, the primary filter is a gaseous removal filter for removing the gaseous pollutant and the secondary filter is a particulate removal filter for removing the particulate pollutants. The turning on of the air flow path adjuster will enable the air to bypass the primary filter, the turning off of the air flow path adjuster will enable the air to pass through the primary filter. In any event the air would pass through the secondary filter.

In one embodiment, when the concentration of particulates pollutants in the air is higher than the designated, or default value, by adjusting the airflow rate of the fan, and by adjusting the position of the air flow path adjuster to an opened position, the air flow will bypass the primary filter. The airflow rate is flowing within the air purification device and be discharged at a velocity A.

In one embodiment, when the concentration of gaseous pollutant in the air is above a designated default value, by adjusting the airflow rate of the fan, and by adjusting the position of the air flow path adjuster to a closed position, and let the device to operate at default flow rate, The airflow rate is flowing within the air purification device and be discharged at a velocity B.

I one embodiment, the velocity A is faster than the velocity B.

In one embodiment, when the gaseous pollutants and the particulate pollutant concentrations were higher than the default values, the air flow path adjuster will be moved to an opened position (it is being adjusted to a position so that the air will bypass the primary filter, but only pass through the secondary filter), and at the same time, the air flow rate of the fan is adjusted to accelerate(Velocity A); when the level of the particulates reach to a desired level, the air flow path adjuster will be turned to a closed position (it is being adjusted so that the air will pass through both the primary and the secondary filter), the airflow of the fan will then be adjusted to operate at a lower default rate, to ensure the gaseous pollutants flow at a lower airflow rate (at a velocity which is lower than Velocity A) and be effectively adsorbed by the primary filter.

In one embodiment, the fan is disposed downstream of the primary filter and the air flow path adjuster, it is disposed upstream of the secondary filter. Further, the air inlet of the fan is connected to the primary filter and the air flow path adjuster. The air outlet of the fan is connected to the secondary filter. When the air flow path adjuster is in a closed position (the air flow path adjuster is being adjusting to a position such that it allow the air to pass through both the primary filter and secondary filter). The air is being drawn into the primary filter, and within the materials of the primary filter, in a laminar flow is formed. Then, the air is blown onto the secondary filter. When the air is drawn to flow through the secondary filter, it is flow in a form of turbulence flow.

In another embodiment, the fan is disposed downstream of both the primary filter and the secondary filter. The air inlet of the fan is connected to the primary filter and the air flow path adjuster, or the secondary filter. Further, when the air flow path adjuster is turned to closed position (it is adjusted to a position so that air can flow through both the primary filter and the secondary filter at the same time), the air is drawn into the primary filter and the secondary filter, the air is flowing as a laminar flow,

In one embodiment, the primary filter is located downstream to the secondary filter.

In one embodiment, the fan is disposed at a position upstream of the primary filter and the secondary filter, the air inlet of the fan is connected to the primary filter and the air flow path adjuster, and / or the secondary filter.

In one embodiment, when the airflow path adjuster is moved to a closed position (or adjusted to a position so that air can flow through both the primary filter and the secondary filter), the air flow of the fan is default to operate at a rate which can withstand an air flow resistance, which shall be at least greater than the sum of the filter static pressure of the primary and the secondary filters.

In one embodiment, when the air flow path adjuster is moved to an opened position (or adjusted to a position so that air can flow through only the secondary filter but not the primary filter), the air flow of the fan is default to operate at a rate which can withstand an air flow resistance, which shall be at least greater than the static pressure of the secondary filter.

In one embodiment, the air purification device further comprising a negative ion generator, the negative ion generator comprises an electronic circuit and a negative ion release tip. The ion releasing tip is provided at an upstream position of the particulate removal filter or the secondary filter.

In one embodiment, the air purification device further comprising a negative ion generator, the negative ion generator comprises an electronic circuit and a negative ion release tip. The ion releasing tip is provided at a position, which the position will in any event, if the air will bypass or pass through the primary filter, the air will anyway pass through the ion releasing tip.

In one embodiment, the primary filter comprises a first filter material layer and the secondary filter material layer for the air to pass through. The first filter material layer is a filter layer for filtering the particulate pollutants, the secondary filter material layer is a filter layer comprises of activated carbon or zeolite-based porous gas adsorbent materials.

In one embodiment, the primary filter further comprises an ultraviolet light and titanium dioxide compounds for catalyze the oxidation and decomposition of organic pollutants in the air.

In one embodiment of the air purification device, the concentration of the particulate pollutants in the air, also referring to the concentration of particulate pollutants in the indoor environment, and/or the concentration of particulate pollutants at the nearby the outdoor environment.

In one embodiment, the air purification device further includes a particulate sensor and a central processing unit, the central processing unit is electrically connected to the fan and the air flow path adjuster. According to the concentration of particulate pollutants and/or gaseous pollutants, the central processing unit control the air flow path adjuster, so that the air flow path adjuster will partially be or completely be opened or closed; the central processor would control the speed of the fan to match the air flow path adjuster.

When the dust sensor detected that the dust in the ambient concentrations is higher than the default value, the central processor will control the air flow path adjuster so that the air will bypass the primary filter and flow directly through the secondary filter; the volatile organic compound function to detect the concentration of the volatile organic compound surrounding the air purification device; Bases on the concentration of volatile organic compounds in the surrounding environment, the time required for such gaseous pollutants to be adsorbed into the gaseous removal filter, and the static pressure that can be withstand by the gaseous removal filter, the central processing unit regulate the airflow speed of the fan.

In one embodiment, the air purification device further bases on the concentration of particulate pollutants and/or gaseous pollutants, the central processing unit to control the air flow path adjuster, so that the air flow path adjuster will partially be or completely be opened or closed; the central processor would control the speed of the fan to match the air flow path adjuster.

In one embodiment, the air flow path adjuster and the primary filter formed integrally into one same component.

In one embodiment, the function of the air flow path adjuster also carry the same function of the primary filter, it has the same or high air flow resistance in compare to the primary filter.

In one embodiment, the secondary filter further comprises an electrostatic precipitator.

In another embodiment of the air purification device, the secondary filter comprises a high efficiency particulate removal filter (HEPA).

When one embodiment, the particulates sensor and or dust sensor is employed to detected the particulate pollutants level at the environment, and further calculate the amount of the particulate could be hold by the filter at the condition on the opening or the closing of the air flow path adjuster. When the cumulative amount of dust exceeded the preset level which could be hold by the filter, the central processing unit of the air purification device gives out warning signal.

The present invention also includes a method of air purification, when the particulates level is higher than a default value, the air flow path adjuster will be opened (or being adjusted to a position so that the air will bypass the primary filter, but flow through the secondary filter), the airflow rate of the fan accelerates. Until the concentration of the particulates reach to a value lower than the default value, the fan will be adjusted to decelerate, and operate based on a manual setting or a default setting automatically. The air flow path adjuster will be opened (or being adjusted to a position so that the air will bypass the primary filter, but flow through the secondary filter) or closed (or being adjusted to a position so that the air will flow through both the primary and the secondary filters).

In the method of air purification, the manual setting comprises a dust removal setting and a volatile organic compound removal setting, if the manual setting is referring to a dust removal setting, when the level of the particulate pollutants in the air is lower than a default value, the airflow rate will be lower down and the air flow path adjuster will remain opened (or being adjusted to a position so that the air will bypass the primary filter, but flow through the secondary filter); if the manual setting is referring to a volatile organic compound removal setting, when the particulate pollutant level in the air is lower than a default value, the air flow path adjuster will be closed (or being adjusted to a position so that the air will flow through both the primary and the secondary filters).

The air purification method, further, if the operation of the air flow path adjuster to opened or closed is based on an automatic setting, the automatic setting should be pre-implemented as an embedded software into the air purification device, or based on the particulates sensor or volatile sensors, or the data on the particulates level, and/or the organic compound level which is obtained from the sensors which is(are) being implemented in the air purification device or off-site of the the air purification device. If the operation is based on the data on the levels of the particulates and/or volatile organic compound which obtained off-site remotely, the data transmission shall be wiring or wireless to the air purification device.

The present patent application described herein are with reference to certain specific embodiments. Without departing from the scope of the present patent application, the present patent application can also be of various changes and/or being substituted by any equivalents. The present patent application can be of various modifications, without departing from the original scope. In addition, the present patent application is not limited to the particular embodiment disclosed herein. Any scope which falling within the scope of the patent claims herein shall be bound by the present invention.

## Claims

1. An air purification device, comprising an air flow path adjuster and a primary filter; the air flow path adjuster means to change the path of the air flow within the air purification device, so that the air either pass through or bypass the primary filter.

2. The air purification device according to claim 1, wherein the primary filter is a filter for removing gaseous pollutants, the air is adjusted to flow at a slower rate when it is to pass through the primary filter, comparing to when it is to bypass the primary filter; or the primary filter is a filter for removing particulate pollutants, the air is adjusted to flow at a faster rate when it is to pass through the primary filter, comparing to when it is to bypass the primary filter.

3. The air purification device according to claim 1, wherein the air purification device further comprises a secondary filter, the primary filter and the secondary filter are placed separately with space.

4. The air purification device according to claim 1, wherein the air flow path adjuster and the primary filter is in a flexible connection; according to a preset sequence, the air first pass through the primary filter and then the secondary filter, the air flow path adjuster is moved or rotated relative to the primary filter, so as to adjust the air flow rate, and enable the air to bypass the primary filter.

5. The air purification device according to claim 1, wherein the air flow path adjuster is connected to the primary filter so that it can be rotational coupled onto the primary filter; the air flow path adjuster overlap on the air-in side of the primary filter, and enable to air to bypass the primary filter.

6. The air purification device according to claim 1, wherein the air flow path adjuster fixedly connected to the primary filter, or the air flow path adjuster and the primary filter is formed integrally by a same materials; the air flow path adjuster and the primary filter rotate integrally to adjust the air flow rate, and enable to air to bypass the primary filter.

7. The air purification device according to claim 1, wherein the air purification device further comprises a fan, the fan is disposed between the primary filter and the secondary filter; the fan enable to air to accelerate and flow from the primary filter or the air flow path adjuster to the secondary filter. The air purification device can adjust the airflow rate of the fan, so as to match the air flow path adjuster.

8. The air purification device according to claim 1, wherein the air purification device further comprises a fan, the fan is disposed adjacent to the primary filter, and away from one side of the secondary filter, or it is disposed to adjacent to the secondary filter, and away from one side of the primary filter; the air purification device can adjust the airflow rate of the fan, so as to match the air flow path adjuster.

9. The air purification device according to claim 1, wherein the fan is capable to generate a negative pressure, so as to enable the air to pass through the secondary filter and the air flow path adjuster as its preset order.

10. The air purification device according to claim 1, wherein the air purification device further comprises a negative ion generator; the ion generator comprises an electronic circuit and an ion releasing tip; and the ion releasing tip is disposed with some space from the primary filter, so as to enable the air be first passing through the ion releasing tip then subsequently the primary filter.

11. The air purification device according to claim 1, wherein the air purification device is formed with one air inlet and two air outlet, the primary filter and the secondary filter are placed at the two air outlets respectively, so as to enable the air be split to pass through the primary filter and the secondary filter at the same time, and the air flow path adjuster is flexibly connected to the primary filter, the air flow path adjuster can be moved relative to the primary filter or turn to rotate to adjust the flow rate of air, and enable to air to bypass the primary filter.

12. The air purification device according to claim 1, wherein the air purification device further comprises a fan, the fan is disposed adjacent to the air inlet, and speed up the air to flow towards the two air outlets; the air purification device can adjust the airflow rate of the fan, so as to match the air flow path adjuster.

13. The air purification device according to claim 1, wherein the air purification device further includes a particulate sensor and a central processing unit, the central processing unit is electrically connected to the fan and the air flow path adjuster; according to the concentration of particulate pollutants and/or gaseous pollutants, the central processing unit control the air flow path adjuster, so that the air flow path adjuster will partially be or completely be opened or closed; the central processor would control the speed of the fan to match the air flow path adjuster.

14. The air purification device according to claim 1, wherein the air purification device further comprises a dust sensor and a volatile organic compound sensor; the dust sensor and the volatile organic compound sensors are connected electrically to the central processing unit; when the dust sensor detected that the dust in the ambient concentrations is higher than the default value, the central processor will control the air flow path adjuster so that the air will bypass the primary filter and flow directly through the secondary filter; the volatile organic compound function to detect the concentration of the volatile organic compound surrounding the air purification device; bases on the concentration of volatile organic compounds in the surrounding environment, the time required for such gaseous pollutants to be adsorbed into the gaseous removal filter, and the static pressure that can be withstand by the gaseous removal filter, the central processing unit regulate the airflow speed of the fan.

15. The air purification device according to claim 1, wherein the primary filter is an electrostatic precipitator, when the cumulative amount of dust exceeded the preset level which could be hold by the primary filter, the central processing unit of the air purification device gives out warning signal.

16. The air purification device according to claim 1, wherein the primary filter and the secondary filter is disposed in a parallel manner, the air pass through the primary filter as a laminar flow, the air pass through the secondary filter as turbulence flow.

17. The air purification device according to claim 1, wherein the primary filter is a gaseous removal filter for removing the gaseous pollutant and the secondary filter is a particulate removal filter for removing the particulate pollutants; or the primary filter is a particulate removal filter for removing the particulate pollutants and the secondary filter is a gaseous removal filter particulate removal filter for removing gaseous pollutant; or the primary filter is for removal of both particulate and gaseous pollutants, whereas the size of the particulate pollutants to be removed by the primary filter will be smaller in compared to that to be removed by the secondary filter.

18. The air purification device according to claim 1, wherein the primary filter is for removing particulate pollutants in a dimension of 2.5 microns or smaller, the secondary filter for removing larger particulate pollutants, which are in a dimension of equal to 10 micron or larger.

19. The air purification device according to claim 1, wherein the secondary filter is an electrostatic precipitator or high efficiency dust filter.

20. The air purification device according to claim 1, wherein the primary filter comprises a first filter material layer and the secondary filter material layer for the air to pass through; the first filter material layer is a filter layer for filtering the particulate pollutants, the secondary filter material layer is a filter layer comprises of activated carbon or zeolite-based porous gas adsorbent materials.

21. The air purification device according to claim 1, wherein the primary filter further comprises an ultraviolet light and titanium dioxide compounds for catalyze the oxidation and decomposition of organic pollutants in the air.
